# EUROPEAN PATENT SPECIFICATION

(11) **EP 1 408 891 B1**
(45) Date of publication and mention of the grant of the patent: **04.10.2006**
(21) Application number: 02776915.7
(22) Date of filing: 24.07.2002
(51) Int. Cl.: A61F 2/44

(54) **DEVICE FOR INTERSOMATIC STABILIZATION USING A MINI-INVASIVE APPROACH**
VORRICHTUNG FÜR DIE INTERSOMATISCHE STABILISIERUNG MIT EINEM MINI-INVASIVEN VERFAHREN
DISPOSITIF POUR STABILISATION INTERSOMATIQUE PEU INVASIVE

(30) Priority: 24.07.2001 IT TO20010723
(43) Date of publication of application: 21.04.2004
(73) Proprietor: Fornari, Maurizio, 20129 Milano (IT)
(72) Inventor: FORNARI, Maurizio, I-20129 Milano (IT)
(74) Representative: Lotti, Giorgio
(86) International application number: PCT/EP2002/008238
(87) International publication number: WO 2003/009786

(56) References cited:
- WO-A-01/13807
- DE-U- 29 712 331
- FR-A- 2 742 044
- FR-A- 2 764 795
- US-A- 5 458 638
- US-A- 6 056 749
- US-A- 6 093 207

## Description

The present invention relates to a device for intersomatic stabilization using a mini-invasive approach designed to be inserted between two contiguous vertebrae for keeping the two vertebrae at a distance apart from one another and for favouring their intersomatic fusion.

In degenerative discopathies and other similar conditions that lead to the collapse of an intervertebral disc with a consequent vertebral instability and co-presence of backache, there is known the use of devices for intersomatic stabilization designed to reconstitute the intervertebral space and to restore, simultaneously, spinal stability, which is fully obtained following upon completion of a valid bone fusion between the two vertebrae concerned. A device of that kind is known from US 6 093 207 A.

Known devices for intersomatic stabilization, which are generally referred to as "cages", may be of the screwed type or else of the impact type and entail the use of further tools for the preparation of a threaded seat for the cage and for maintaining the correct intervertebral distance during insertion of the device itself, at the end of which preparatory step a certain amount of homologous or autologous bone is normally inserted for the purpose of facilitating intersomatic fusion.

Once fusion is achieved, the two contiguous vertebrae are perfectly stable, and the implanted cage is completely integrated with attainment of spinal stability, as well as disappearance of pain.

Known surgical techniques resort to different modes of access for the implantation of the above-mentioned devices for intersomatic stabilization. Various modes of access are in fact practicable: posterior, lateral or anterior. These are chosen according to the particular condition to be treated and the inclinations and modes of operating of the surgeon.

Devices for intersomatic stabilization of the type described above and the possibility afforded by them for being inserted only through the customary routes of access pose a number of problems, which are due also to the dimensions of the devices normally commercially available.

In fact, the above devices may cause a significant destruction of the bone stock, both at the level of the compact bone of the discs and at the level of the laminae and of the articular surfaces. Added to this is the further negative aspect that the two contiguous vertebral bodies, which are disadvantageously damaged in their integrity, both anterior and posterior, on account of the intervention of preparation and insertion of the device, also lose their intrinsic stability.

The purpose of the present invention is to provide a device for intersomatic stabilization using a mini-invasive approach, which presents structural and functional characteristics such that the implantation proves significantly less invasive and, especially, such as to guarantee the almost absolute integrity of laminae and articular processes, thus solving the problems described above.

According to the present invention, there is provided a device for intersomatic stabilization using a mini-invasive approach, which is designed to be inserted between two contiguous vertebrae to keep them at a distance from one another and which comprises a substantially cylindrical body extending along a longitudinal axis and a thread external to and fixed to the body itself. The said device is characterized in that it comprises a head end, which is fixed to the body and defined by an elliptical cross section that increases along the axis, the end being designed to dilate the intervertebral space as advance of the end itself proceeds in the intervertebral space until there is created by the thread a seat for the subsequent advance of the device with stabilizing function.

The invention will now be described with reference to the attached drawings, which illustrate a non-limiting example of embodiment thereof and in which:
- Figure 1 is a rear perspective view of a first preferred embodiment of the device for intersomatic stabilization using a mini-invasive approach according to the present invention;
- Figure 2 is a front perspective view of the device of Figure 1;
- Figure 3 is a cross-sectional view of the device of Figure 1 taken according to an axial plane of the device itself.

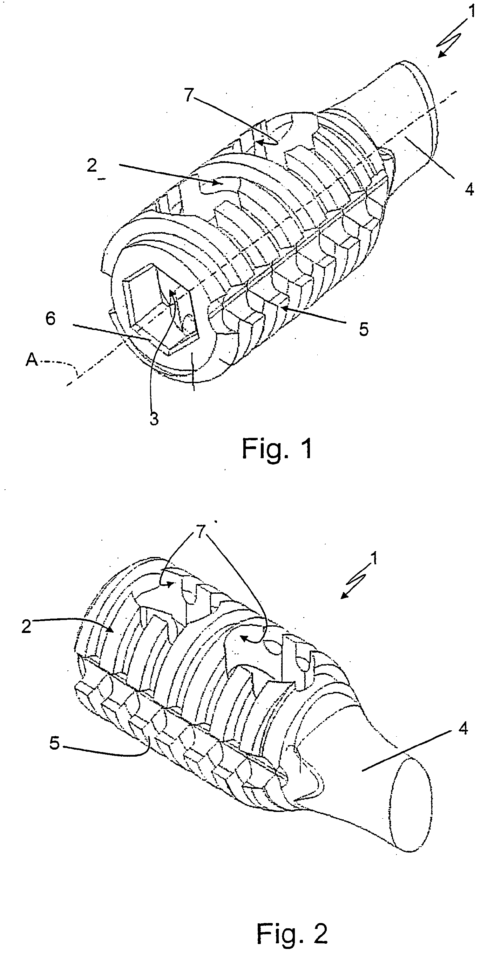

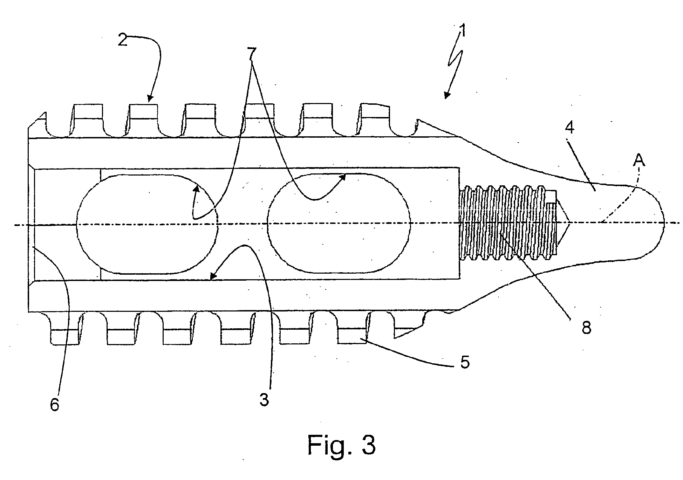

With reference to Figures 1, 2 and 3, the reference number 1 designates as a whole a device for intersomatic stabilization using a mini-invasive approach designed to be inserted between two contiguous vertebrae for keeping the two vertebrae themselves at a distance apart from one another and for favouring their subsequent intersomatic fusion.

The device 1 comprises a hollow cylindrical body 2, which extends along a respective major longitudinal axis A and is provided with an internal through cavity 3, and a shaped head 4 set at one end of the body 2. The body 2 is moreover provided with a threaded external surface 5, the thread possibly having even more than one start, which extends throughout the body 2 itself starting from the head 4 and enables screwing of the device 1 in the space between the two vertebrae referred to above.

The cavity 3 presents, in a cross section transverse to the axis A, a substantially cylindrical shape, and is provided with a slot 6 with a hexagonal cross section defining a seat for transmission of the tightening torque to the device 1 by a manoeuvring tool, as well as with a further seat 8 set along the axis A and inside the head 4, the said seat 8 being designed to cause the device 1 and the above-mentioned manoeuvring tool to be integrally fixed together.

The cavity 3 is moreover provided with four through holes 7, which are made through the surface 5 to set the cavity 3 in communication with the outside world and are aligned in twos along the axis A and aligned in twos in a direction transverse to the axis A.

The surface 5 has an external diameter that is practically constant along the entire axis A, except for the area immediately adjacent to the slot 6, and the area immediately contiguous to the head 4, which, instead, is tapered towards the head 4 itself and is made in such a way as to be self-tapping, according to technologies that are already known and applied in mechanical engineering.

The head 4 has the task, during insertion of the device 1 between the aforementioned two vertebrae, of appropriately dilating the intervertebral space so as to enable the subsequent screwing of the device 1 itself, and is defined by a solid of revolution of increasing section having a substantially elliptical cross section and a thickness S, which varies as the angular position of the device 1 about the axis A varies.

The head 4 is made so as to enable its insertion in an intervertebral space of limited height and then, by means of a rotation through 90 degrees, to increase the intervertebral space itself up to the height necessary for the surface 5 to be able to start gripping in the bone of the aforementioned two vertebrae, creating in the bone itself a seat for the subsequent advance of the device 1 having stabilizing function.

The device 1 described above renders unnecessary both the dilators used before the introduction of all the cages mentioned above in the introduction and the positioning and maintenance in situ of dilators or spacers that guarantee maintenance of the intersomatic distance in the step of milling and threading of the vertebral bodies prior to introduction of the threaded cages.

The resultant of these two advantages is the availability of a cage having a dilating head 4, which guarantees, during the step of tapping, a correct spontaneous positioning of the head 4 itself between the two vertebral bodies, the said relative distance being maintained as the advance of the head 4 of the body 2 proceeds.

In other words, the device 1 can enable provision of an intersomatic arthrodesis with posterior or monolateral intraforaminal bilateral mini-invasive approach, and, finally, positioning by posterior or posterolateral (intraforaminal) route of the device 1 will not cause any destruction or destabilization of the posterior compartment, providing, instead, an extremely valid intersomatic arthrodesis.

It is to be understood that the invention is not limited to the embodiments described and illustrated herein, which are to be considered as examples of embodiment of the device for intersomatic stabilization using a mini-invasive approach, the said device being liable to further modifications as regards the shapes and arrangement of parts, and as regards details of construction and assembly.

## Claims

1. A device (1) for intersomatic stabilization using a mini-invasive approach designed to be inserted between two contiguous vertebrae to keep them at a distance from one another, and comprising:
- a substantially cylindrical body (2), which extends along a longitudinal axis (A);
- a thread (5) external to and fixed to the body (2) itself; and
- a head end (4), which is fixed to the body (2) and has a cross section that increases along the axis (A);
the device (1) being charactherized by the fact that said head end (4) has an elliptical cross section so as to present a thickness, which varies as an angular position of the body (2) about the said axis (A) varies, the head end (4) being designed to dilate the intervertebral space as advance of the end (4) itself proceeds in the intervertebral space until there is created with the thread (5) a seat for the subsequent advance of the device (1) having stabilizing function.

2. The device according to Claim 1, **characterized in that** said head end (4) has an increasing oval cross section so as to dilate the intervertebral space as its advance in the intervertebral space proceeds.

3. The device according to Claim 1, **characterized in that** said body (2) is provided with seat (6) with a hexagonal cross section for angular coupling of the device (1) itself with a manoeuvring tool.

4. The device according to Claim 3, **characterized in that** said body (2) comprises four through holes (7), which are made through said thread (5) to set an internal cavity (3) of the body (2) in communication with the outside world, and which are aligned in twos along the axis (A) and aligned in twos in a direction transverse to the axis (A) itself.

## Patentansprüche

1. Vorrichtung (1) für die intersomatische Stabilisierung mit einem miniinvasiven Verfahren, dazu ausgelegt, zwischen zwei benachbarte Wirbel eingeführt zu werden, um diese in einem Abstand zueinander zu halten, und umfassend:
- einen im Wesentlichen zylindrischen Körper (2), der sich entlang einer Längsachse (A) erstreckt;
- ein Gewinde (5) außerhalb des und an dem Körper (2) selbst befestigt; und
- ein Kopfende (4), das an dem Körper (2) befestigt ist und einen Querschnitt hat, der entlang der Achse (A) zunimmt;
wobei die Vorrichtung (1) **dadurch gekennzeichnet ist, dass** besagtes Kopfende (4) einen elliptischen Querschnitt hat, um eine Dicke aufzuweisen, die in dem Maße variiert, in dem eine Winkelposition des Körpers (2) um die besagte Achse (A) herum variiert, wobei das Kopfende (4) dazu ausgelegt ist, den Zwischenwirbelraum zu weiten, während das Vordringen des Endes (4) selbst im Zwischenwirbeiraum fortschreitet, bis mit dem Gewinde (5) ein Sitz für das anschließende Vordringen der eine stabilisierende Funktion aufweisenden Vorrichtung (1) geschaffen wurde.

2. Die Vorrichtung nach Anspruch 1; **dadurch gekennzeichnet, dass** besagtes Kopfende (4) einen zunehmenden ovalen Querschnitt aufweist, damit es mit seinem fortschreitenden Vordringen im Zwischenwirbelraum den Zwischenwirbelraum weitet.

3. Die Vorrichtung nach Anspruch 1, **dadurch gekennzeichnet, dass** besagter Körper (2) versehen ist mit Sitz (6) mit einem sechseckigen Querschnitt für das winkelige Ankoppeln der Vorrichtung (1) selbst mit einem Manövrierwerkzeug.

4. Die Vorrichtung nach Anspruch 3, **dadurch gekennzeichnet, dass** besagter Körper (2) vier Durchgangslöcher (7) aufweist, die durch besagtes Gewinde (5) angebracht sind, um einen internen Hohlraum (3) des Körpers (2) in Verbindung mit der Außenwelt zu bringen, und die paarweise entlang der Achse (3) ausgerichtet sind und paarweise in einer Richtung quer zu der Achse (A) selbst ausgerichtet sind.

## Revendications

1. Dispositif (1) pour stabilisation intersomatique utilisant une approche mini-invasive conçu pour être inséré entre deux vertèbres contiguës afin de les garder à distance l'une de l'autre, et comprenant :
- un corps (2) sensiblement cylindrique qui s'étend le long d'un axe longitudinal (A) ;
- un filet (5) externe et fixé au corps (2) proprement dit ; et
- une extrémité de tête (4) qui est fixée au corps (2) et qui a une coupe transversale qui augmente le long de l'axe (A) ;
le dispositif (1) étant **caractérisé par le fait que** ladite extrémité de tête (4) a une coupe transversale elliptique afin de présenter une épaisseur qui varie en même temps qu'une position angulaire du corps (2) autour dudit axe (A) varie, l'extrémité de tête (4) étant conçue pour dilater l'espace intervertébral en même temps que s'effectue la progression de l'extrémité (4) proprement dite dans l'espace intervertébral jusqu'à ce qu'il se crée avec le filet (5) un siège pour la progression ultérieure du dispositif (1) remplissant une fonction de stabilisation.

2. Dispositif selon la revendication 1, **caractérisé en ce que** ladite extrémité de tête (4) a une coupe transversale ovale croissante afin de dilater l'espace intervertébral en même temps que s'effectue sa progression dans l'espace intervertébral.

3. Dispositif selon la revendication 1, **caractérisé en ce que** ledit corps (2) est doté d'un siège (6) ayant une coupe transversale hexagonale pour un couplage angulaire du dispositif (1) proprement dit avec un outil de manipulation.

4. Dispositif selon la revendication 3, **caractérisé en ce que** ledit corps (2) comprend quatre trous traversants (7) qui sont réalisés au moyen dudit filet (5) pour faire communiquer une cavité interne (3) du corps (2) avec le monde extérieur et qui sont alignés par deux le long de l'axe (A) et qui sont alignés par deux dans une direction transversale à l'axe (A) proprement dit.
